Europäisches Patentamt

European Patent Office

Office européen des brevets

(19)

(11) Publication number: **0 384 098**

**A1**

(12) # EUROPEAN PATENT APPLICATION

(21) Application number: **89850068.1**

(22) Date of filing: **24.02.89**

(51) Int. Cl.⁵: **A61B 17/16**

(43) Date of publication of application:
**29.08.90 Bulletin 90/35**

(84) Designated Contracting States:
**CH DE FR GB IT LI NL SE**

(71) Applicant: **Odensten, Magnus**
**Hjulsbrovägen 104**
**S-582 69 Linköping(SE)**

Applicant: **Gillquist, Jan**
**Stjärnorpsvägen Berg**
**S-590 61 Vreta Kloster(SE)**

(72) Inventor: **Odensten, Magnus**
**Hjulsbrovägen 104**
**S-582 69 Linköping(SE)**
Inventor: **Gillquist, Jan**
**Stjärnorpsvägen Berg**
**S-590 61 Vreta Kloster(SE)**

(74) Representative: **Westerlund, Christer et al**
**L.A. Groth & Co Patentbyra AB**
**Västmannagatan43**
**S-113 25 Stockholm(SE)**

(54) **A cruciate ligament surgical drill guide.**

(57) A posterior cruciate ligament surgical drill guide adapted to be temporarily attached to a human knee to guide a drill rod drilling a channel for receiving a portion of a posterior cruciate ligament substitute to extend between the tibia and the medial femoral condyle, the drill guide comprising a handle (1,2), a first guiding element (6) connected (4) to the handle (1,2) and adapted to be positioned externally of the tibia and obliquely relative the longitudinal axis thereof and a second guiding element (11) connected (9) to the handle (1,2) and adapted to be positioned externally of the femur and obliquely relative the longtudinal axis thereof, the orientations of the first (6) and second (11) guiding elements being such that the locations on the tibial plateau surface and on the inner surface of the medial femoral condyle, respectively, where the mouths (8) of the channels coaxial with the longitudinal axes of the guiding elements (6,11) are situated, will correspond to the attachment points of the posterior cruciate ligament.

Fig.1

## A cruciate ligament surgical drill guide

### FIELD OF THE INVENTION

The present invention refers to a surgical tool, particularly a drill guide for human knee surgery. The drill guide is adapted to be temporarily attached to a human knee to guide a drill rod drilling a channel for receiving a portion of a posterior cruciate ligament to extend between the tibia and the medial femoral condyle.

When carrying out a surgical operation in which a posterior cruciate ligament substitute is to be inserted in the tibia and in the femur, it is essential that the ligament substitute extends between the attachment points of a normal human cruciate ligament to ensure that the knee will function satisfactorily for all possible angles between the femur and the tibia and that the stresses on the ligament substitute and its fastening means will be as small as possible.

### BACKGROUND ART

Several drill guides have been proposed which guide drill rods so that the drilled channels will open within the knee at the attachment points of normal cruciate ligaments. The most common of such drill guides are C-guides which may be used for surgical operation of anterior cruciate ligaments as well as posterior cruciate ligaments, see for instance US patent No. 4 257 411 and the references cited therein. The main disadvantage of such guides is that two separate drill guides must be used or one single drill guide must be used twice for drilling both channels. This is not only costly and time-consuming but requires separate alignment and setting of each guide implying that the relationship between the drilled channels as to distances and angles between them will not be as exact as required.

A drill guide for simultaneously drilling two channels for an anterior cruciate ligament substitute has also been proposed, see Swedish patent No. 84 01427-3. However, this guide cannot be used for drilling the channels for a posterior cruciate ligament substitute because such channels cannot be in alignment with each other and because the attachment points for a posterior cruciate ligament are not isometrically positioned.

### SUMMARY OF THE INVENTION

Studies have been made to locate the attachment points of human cruciate ligaments and to determine the best attachment points of human cruciate ligament substitutes. It was found that the average length of the posterior cruciate ligament for adults was 38 +/-4 mm and that the average angle between the ligament and the longitudinal axis of the femur was 43.3 +/-3 degrees with the knee in full extension. It was also found that the best location for a ligament substitute was between the central point of the normal attachment area on the tibia and the central proximal part of the femoral attachment area on the medial femoral condyle because the distance between said central point and said proximal part was found to change the least during the full range of knee motion, thus minimizing wear and stresses on the ligament substitute and the bone parts to which it is fastened.

On the basis of these findings, a drill guide according to the present invention was designed which permits the two channels in the tibia and in the femur to be drilled by means of one single instrument and at a predetermined distance and angle between them so that the inner mouths of the channels will be positioned at the central point of the normal attachment area on the tibia and the central promixal part of the femoral attachment area on the medial femoral condyle, respectively, and the channels will be directed to minimize bending and torque of a ligament substitute extending between the attachment points.

### BRIEF DESCRIPTION OF THE DRAWINGS

Fig. 1 is a lateral view of a drill guide according to an embodiment of the present invention attached to a human right knee, and

Fig. 2 is an end view of the drill guide as seen from the left in Fig. 1.

### DETAILED DESCRIPTION OF A PREFERRED EMBODIMENT

Before describing the drill guide of the present invention it seems appropriate to give further details about the anatomy of the human knee, especially with respect to the posterior cruciate ligament as found in the studies referred to above.

The total length (mid-point distance) of the posterior cruciate ligament was found to be 38 +/-4 mm. The thickness was 6 +/-1 mm and the width 14 +/-2 mm, both measured in the middle third of the ligament. The ligament consists of three different bundles of fibres oriented in different directions. Only the fibres originating from the most posterior central part of the femur attachment fulfill the criteria of isometricity.

The average angle between the longitudinal axis of the ligament and the longitudinal axis of the femur with the knee at 0° of flexion was 43+/-3°. When the knee is fully extended, the ligament runs in a line parallell to the posterior slope of the tibial spine.

The area of the attachment of the posterior cruciate ligament on the medial femoral condyle was oval. Its maximum diameter was 20+/-2 mm and its lesser diameter 10+/-2 mm. The distance from the central point of the attachment area to the junction between the posterior area of the femoral axis and the proximal surface of the lateral femoral condyle was 25+/-3 mm.

The area of the tibial attachment was more or less a square. The length of this area was 14+/-1 mm and its width 12+/-2 mm. The attachment area was located on the posterior slope of the tibial spine anterior to the posterior edge of the tibial plateau.

The most favourable insertion point regarding changes in ligament length was located in the central promixal part of the femoral attachment area. The ligament length increased by 0.9+/-1.5 mm at 60° of knee flexion.

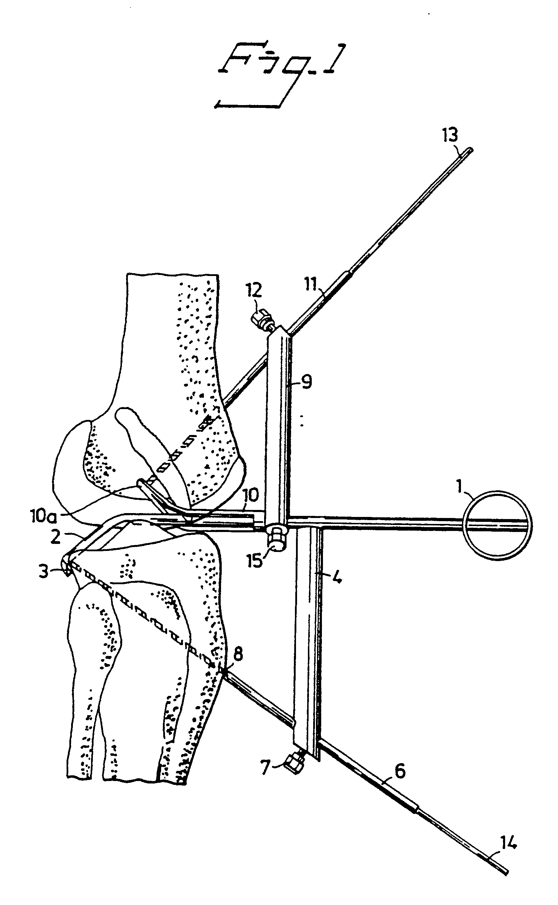

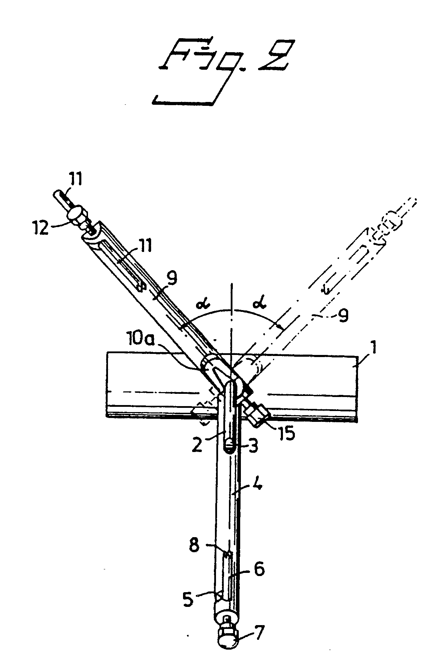

The drill guide of the present invention consists of a metal frame or handle. The handle is provided with a tubular hand grip 1 and a rod 2, the end of which remote from the hand grip 1 being bent downwards in Figs. 1 and 2 and terminating in a hook 3. A cylindrical holder 4 is connected and extends perpendicularly to the rod 2. The end of the holder 4 remote from the rod is provided with a boring 5 in which a guide tube 6 is inserted. The guide tube 6 may be displaced in the boring 5 and locked in any position relatively to the holder 4 by a screw 7. One end of the guide tube 6 is provided with a sawtooth edge 8. The angular orientation of the guide tube 6 is such that the imaginary extension thereof will intersect the end of the rod 2 immediately above the hook 3 in Fig. 1. The angle between the guide tube 6 and the straight portion of the rod 2 is in the range of 20° - 45°, suitably 30°.

A second cylindrical holder 9 extends perpendicularly to the rod 2 and is supported thereon by means of a boring in the holder 9 such that the holder may be rotated on the rod and displaced in the longitudinal direction of the rod. A screw 15 on the holder 9 is used to set the holder on the rod 2 so that the holder will be directed at an angle α of approximately 43° to the holder 4. In Fig. 2, the holder 9 is shown with full lines angled to the left on the rod. This position is used when the drill guide is used for a right knee. The position of the holder 9 shown with dashed lines in Fig. 2 is the one used when the drill guide is used for a left knee. In the latter position, the holder 9 forms the same angle α to the holder 4 as in the first position.

A slitted tube 10 surrounds the rod 2 and is fastened to the holder 9. The end of the tube 10 remote from the holder 9 is bent (at 10 a) in the same plane as that of the holder 9 to lie on the imaginary extension of a guide tube 11 inserted in a boring in the holder 9. The guide tube 11 has the same design as the guide tube 6 and may be set to different axial positions and locked therein by a screw 12. The guide tube 11 forms an angle with the rod 2 which is in the range of 25° - 50° preferably 40°.

The use of the drill guide of the invention will now be described.

First the remnant of the posterior cruciate ligament and also the posterior capsule is carefully removed from the posterior slope of the tibial spine through the notch between the femur condyles. The drill guide is then introduced through the notch and the hook 3 of the rod 2 is placed in the posterior sulcus of the tibia. The knee is then extended fully and the rod 2 of the drill guide is held perpendicularly to the longitudinal axis of the leg. The two adjustable guide tubes 6 and 11 are fixed to the anterior part of the tibia and to the fronto-medial part of the medial femoral condyle just outside the joint capsule, respectively. The femoral guide tube holder 9 is adjusted to the right or left knee by angulation relatively to the handle of the drill. Guide pins 13 and 14 are drilled through the femur and the tibia while guided by the guide tubes 6 and 11. The end of the rod 2 near the hook 3 and the bent end 10a of the tube 10 function as drill rod stopping elements to prevent nerve and vessel injury in the posterior portions of the tibia and femur.

The inner mouths of the channels drilled by the guide pins 13 and 14 will be at a distance from each other of approximately 38 mm, and the angle between a line connecting these mouths and the longitudinal axis of the femur will be approximately 43°.

The guide tubes 6 and 11 and the drill guide are then removed. The guide pins are then overdrilled with a cannulated drill (not shown) of desired size and the mouths of the channels are bevelled.

By means of the drill guide according to the invention, the risk of drift and wear of prostetic ligament substitutes at the attachments is minimized. The substitute follows a straight line through the tibia joint space and femur in the frontal plane but for obvious reasons not in the lateral plane. It has been shown that torque in the substitute is generated by channels deviating laterally in the frontal plane. Channels of this type are at an angle to the intraarticular portion of the substitute in both planes and will produce both bending and torque at the attachments which may lead to breakage. With the present drill guide this problem will be reduced. Both the tibial and femoral channels are placed close to a straight line in the frontal plane, and torque in the substitute is minimized.

Although only one embodiment of the drill guide according to the present invention has been described above and shown on the drawings, it should be understood that the invention is not limited to this embodiment but only by the claims.

**Claims**

1 A posterior cruciate ligament surgical drill guide adapted to be temporarily attached to a human knee to guide a drill rod drilling a channel for receiving a portion of a posterior cruciate ligament substitute to extend between the tibia and the medial femoral condyle, the drill guide comprising a handle, a first guiding element connected to the handle and adapted to be positioned externally of the tibia and obliquely relative the longitudinal axis thereof and a second guiding element connected to the handle and adapted to be to positioned externally of the femur and obliquely relative the longitudinal axis thereof, the orientations of the first and second guiding elements being such that the locations on the tibial plateau surface and on the inner surface of the medial femoral condyle, respectively, where the mouths of the channels coaxial with the longitudinal axes of the guiding elements are situated, will correspond to the attachment points of the posterior cruciate ligament.

2 A drill guide as claimed in claim 1 wherein the second guiding element is mounted on the drill guide to be set in anyone of two different positions, one position to guide a drill rod for drilling a channel through the medial femoral condyle on a right knee and another position to guide a drill rod for drilling a channel through the medial femoral condyle on a left knee.

3 A drill guide as claimed in claim 1 wherein a first drill rod stopping element is provided on a portion of the drill guide to be introduced in the knee, the first stopping element being at a distance from the first guiding element slightly exceeding the length of the channel in the tibia and intersecting the imaginary extension of the longitudinal axis of the first guiding element.

4 A drill guide as claimed in claim 3 wherein said drill guide portion is provided with a means adapted to bear against the sulcus of the tibia.

5 A drill guide as claimed in claim 1 wherein a second drill rod stopping element is provided on a portion of the drill guide to be introduced in the knee, the second stopping element being at a distance from the second guiding element slightly exceeding the length of the channel in the femoral condyle and intersecting the imaginary extension of the longitudinal axis of the second guiding element.

6 A drill guide as claimed in claims 2 and 5 wherein said second stopping element is connected to the second guiding element to follow the settings thereof.

7 A drill guide as claimed in claim 1 wherein the angle between the first guiding element, lying in a sagittal plane including the longitudinal axis of the femur when the drill guide is in use with the knee in full extension, and the second guiding element, lying in a plane intersecting the sagittal plane, is between $40°$ and $47°$, preferably $43°$.

Fig.1

Fig. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 253 688 (LABOUREAU) <br> * Abstract; column 3, line 40 - column 5, line 38; figures * <br> --- | 1,3-6 | A 61 B 17/16 |
| A,D | EP-A-0 162 027 (ODENSTEN) <br> * Abstract; figures * <br> --- | 1 | |
| A | FR-A-1 505 998 (FRAIN) <br> * Page 1, column 2, line 21 - page 2, column 1, line 35; figures 1,2,6 * <br> --- | 1 | |
| A | US-A-4 739 751 (SAPEGA) <br> * Whole document * <br> --- | 1,3,5 | |
| A | DE-U-8 702 208 (HOWMEDICA) <br> --- | | |
| A | EP-A-0 265 659 (PURNELL) <br> --- | | |
| A | US-A-4 257 411 (CHO) <br> --- | | |
| A | EP-A-0 081 857 (DAWIDOWSKI) <br> ----- | | TECHNICAL FIELDS SEARCHED (Int. Cl.5) <br><br> A 61 B |

The present search report has been drawn up for all claims

| Place of search | Date of completion of the search | Examiner |
|---|---|---|
| THE HAGUE | 09-10-1989 | KLEIN C. |